(19) Europäisches Patentamt
European Patent Office
Office européen des brevets

(11) Numéro de publication: **0 023 214**
**B1**

(12) # FASCICULE DE BREVET EUROPEEN

(45) Date de publication du fascicule du brevet: **09.02.83**

(51) Int. Cl.³: **C 12 P 13/04**, C 12 N 9/80

(21) Numéro de dépôt: **80900241.3**

(22) Date de dépôt: **24.01.80**

(86) Numéro de dépôt international: **PCT/FR 80/00008**

(87) Numéro de publication internationale: **WO 80/01571 (07.08.80** Gazette **80/18)**

(54) **PROCEDE DE PREPARATION D'ACIDES ALPHA-AMINES OPTIQUEMENT ACTIFS PAR HYDROLYSE BIOLOGIQUE DE NITRILES ET PRODUITS OBTENUS.**

(30) Priorité: **24.01.79 FR 7901803**

(43) Date de publication de la demande: **04.02.81 Bulletin 81/5**

(45) Mention de la délivrance du brevet: **09.02.83 Bulletin 83/6**

(84) Etats contractants désignés: **CH DE NL**

(56) Documents cités:
**FR-A-2 245 585**
**FR-A-2 294 999**
**FR-A-2 337 761**
**Chemical Abstracts, volume 89, No. 19, November 1978 Columbus, Ohio U.S. abstact 161521y, page 417**

(73) Titulaire: **ANVAR Agence Nationale de Valorisation de la Recherche, 13, rue Madeleine Michelis, F-92522 Neuilly-sur-Seine (FR)**

(72) Inventeur: **JALLAGEAS, Jean-Claude, "Le Maurétania", rue Triolet, 34000 Montpellier (FR)**
Inventeur: **ARNAUD, Alain, 35, rue Nationale, 34800 Clermont L'Herault (FR)**
Inventeur: **GALZY, Pierre, "Les Moulins" Avenue des Moulins, 34000 Montpellier (FR)**

(74) Mandataire: **Martin, Jean-Jacques et al, Cabinet REGIMBEAU 26, Avenue Kléber, F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

# Procédé de préparation d'acides $\alpha$-aminés optiquement actifs par hydrolyse biologique de nitriles et produits obtenus

La présente invention concerne un procédé de préparation d'acides $\alpha$-aminés optiquement actifs par hydrolyse biologique des $\alpha$-amino-amides correspondants ou des $\alpha$-amino-nitriles correspondants.

Dans la technique antérieure on prépare les acides $\alpha$-aminés optiquement actifs en général à partir de l'amino-acide racémique par dédoublement des stéréoisomères, ceci par des techniques connues, en particulier par formation de sels avec d'autres composés optiquement actifs ou bien par la mise en oeuvre de résines certaines caractéristiques stéréospécifiques.

Le brevet français 2 337 761 décrit la préparation de L-$\alpha$-amino-acides à partir des amides racémiques correspondants en utilisant des procédés microbiologiques.

Le brevet français 2 245 585 décrit la préparation d'amides racémiques à partir des nitriles correspondants pour obtenir ensuite l'amino-acide correspondant.

Mais, aucun de ces brevets ne suggère la possibilité d'obteir directement un amino-acide optiquement actif en partant de l'amino-nitrile racémique, ce qui constitue précisément l'objet de la présente invention.

Outre le fait que ces techniques sont assez délicates de mise en oeuvre, elles imposent, bien entendu, une étape supplémentaire lors de la synthèse des amino-acides à partir d'autres produits tels que les amino-amides correspondants ou les amino-nitriles correspondants qui sont, en général, les précurseurs normaux de ces amino-acides.

Pour ce faire, la présente invention concerne un procédé de préparation de L-$\alpha$-amino-acide à partir de l'$\alpha$-amino-nitrile racémique correspondant sous forme libre ou sous forme de sel, caractérisé en ce que l'on hydrolyse, en une seule étape, en milieu liquide, l'$\alpha$-amino-nitrile racémique correspondant sous forme libre ou sous forme de sel à l'aide d'une bactérie possédant une nitrilase générale et une amidase L-stéréospécifique ou une préparation acellulaire extraite de cette bactérie et en ce que l'on sépare le L-$\alpha$-amino-acide obtenu du D-$\alpha$-amino-amide non hydrolysé.

Dans ce que va suivre, on entend désigner par »amino-amide« ou »amino-nitrile« ou »amino-acide«, aussi bien les composés sous forme libre que sous forme de sel, en particulier de chlorhydrate.

Les agents utilisés dans le cadre du procédé selon la présente invention sont, de préférence, des bactéries ou des préparations acellulaires d'origine bactérienne ne possédant pas d'amidase générale.

Dans le cas où l'$\alpha$-amino-acide est préparé à partir de l'$\alpha$-amino-nitrile correspondant, il est particulièrement intéressant que la bactérie ou la préparation acellulaire d'origine bactérienne possède à la fois l'amidase L-stéréospécifique et la nitrilase générale de façon à n'avoir à utiliser qu'une seule souche ou préparation acellulaire d'origine bactérienne.

Les bactéries ou les préparations acellulaires d'origine bactérienne proviennent, de préférence, d'une souche mutante d'une souche possédant elle-même une amidase générale, ledit mutant étant capable de croître sur un milieu nutritif contenant du monofluoro-acétamide par perte de l'amidase générale.

En effet, on a constaté que lorsque l'on fait croître, sur un milieu nutritif contenant du monofluoro-acétamide, des souches possédant une amidase générale, celles-ci transforment le monofluoro-acétamide en acide monofluoro-acétique selon la réaction:

$$FCH_2CONH_2 \rightarrow FCH_2COOH$$

lequel est toxique pour ces bactéries, ce qui a évidemment pour effet de faire apparaître des mutants spontanés défectifs, c'est-à-dire ne possédant plus l'amidase générale mais uniquement l'amidase L-Stéréospécifique.

Les mutants obtenus dans ces conditions sont des mutants spontanés mais, bien entendu, il est possible d'utiliser des agents mutagènes connus tels que des rayonnements ultraviolets, X ou $\gamma$ ou des composés chimiques tels que l'éthylméthanesulfonate, l'acide nitreux, les agents alkylants, la nitrosoguanidine, l'acryflavine par exemple, afin d'augmenter la fréquence d'apparition des souches possédant l'amidase L stéréospécifique.

Parmi les souches possédant une amidase générale, il faut citer plus particulièrement les genres Bacillus, Bacteridium au sens de Prévot, Micrococcus et Brevibacterium au sens de Bergey. De façon encore préférable, ces bactéries sont choisies parmi les souches de la collection de la chaire de génétique et microbiologie de l'Ecole Nationale Supérieure Agronomique de Montpellier (R 332, R 340, R 341, A 111, B 222, A 112, A 13, A 141, A 142, B 211, B 212, B 221, C 211, R 21, R 22, R 311, R 312, R 331 ou parmi celles déposées au Centraalbureau voor Schimmelcultures sous les numéros: 717-73, 494-74, 495-74, 496-74, 497-74, 498-74, 499-74.

Ces souches possédant une amidase générale présentent les caractéristiquescommunes suivantes:

— Gram positif; alcoolo-acido résistance négative.
— Aérobiose stricte; catalase positive.

- Utilisation du glucose, du saccharose, du maltose et du lactose par voie oxydative sans production de gaz et sans acidification.
  Aucune souche ne forme d'alcool. L'amidon n'est pas hydrolysé mais il y a croissance sur pomme de terre.
- Test de recherche de la tyrosinase sur pomme de terre négatif.
- Besoin en vitamines.
- Absence d'hydrolyse de la gélatine.
- Croissance sur ammoniaque et sur nitrates comme seule source d'azote.
- Pas de dégagement d'hydrogène sulfuré.
- Absence de croissance en présence de bouillon hypersalé.

Tableau I

Caracteristiques Morphologiques

| Souche | Spore | Mobilité | Morphologie cellulaire | Morphologie des colonies |
|---|---|---|---|---|
| R 332 | + | faible | Batonnets (1,8–3,6) μ × 0,9 μ | Circulaires, lisses, convexes rosées, à bord entier |
| R 340 | + | – | Batonnets 2,7 μ × 0,9 μ | Circulaires, petites, blanches, à bord diffus |
| R 341 | + | – | Batonnets 2,7 μ × 0,9 μ | Grosses, très granuleuses, blanches, plates |
| A 111 | – | – | Coques 0,9 à 1,8 μ | Circulaires, petites, plissées, convexes, roses à bord lobé |
| B 222 | – | + | Batonnets (3,6–4,5) μ × 0,9 μ | Circulaires, petites, de couleur jaune orangée |
| A 112 | – | faible | Batonnets (1,8–3,6) μ × 0,9 μ | Petites, opaques, en relief, à bord lobé, roses orangées |
| A 13 | – | faible | Batonnets 2,2 μ × 0,9 μ | Circulaires, lisses, opaques, oranges-roses, à bord entier |
| A 141 | – | – | Batonnets (1,8–3,6) μ × 0,9 μ | Petites, presque plates, opaques, granuleuses, oranges-roses, à bord lobé |
| A 142 | – | – | Batonnets (3,6–4,5) μ × 0,9 μ | Circulaires, lisses, opaques, oranges, à bord entier |
| B 211 | – | – | Batonnets 1,8 μ × 0,9 μ | Circulaires, bombées, petites lisses, rosées, à bord entier |
| B 212 | – | – | Batonnets 3,6 μ × 0,9 μ | Circulaires, bombées, lisses, rosées, à bord entier |
| B 221 | – | faible | Batonnets (3,6–4) μ × 0,9 μ | Circulaires, très lobées, en relief, de couleur jaune-orangée |
| C 211 | – | faible | Batonnets (3,6–8,1) μ × 0,9 μ | Circulaires, lisses, brillantes, roses, à bord entier |
| R 21 | – | – | Batonnets 5,4 μ × 0,9 μ | Circulaires, plates, roses granuleuses, à bord légèrement lobé |
| R 22 | – | faible | Batonnets 2,7 μ × 0,9 μ | Circulaires, lisses, oranges, en relief, à bord entier |
| R 311 | – | faible | Batonnets (1,8–3,6) μ × 0,9 μ | Circulaires, jaunes, en relief, à bord entier |
| R 312 | – | – | Batonnets (4,5–9) μ × 0,9 μ | Circulaires, convexes, jaunes à bord entier |
| R 331 | – | – | Batonnets 4,5 μ × 0,9 μ | Circulaires, roses, plates diffuses et opaques |

Tableau II

Principaux caracteres physiologiques

| Souche | Test oxydase | Indole | Utilisation acide citrique | Hydrolyse du blanc d'oeuf | pH Optimum | Production acétyl-méthylcarbinol |
|---|---|---|---|---|---|---|
| R 332 | – | – | – | – | 6,5 | forte |
| R 340 | – | + | + | – | 6,5 | – |
| R 341 | – | + | – | – | 6,0 | – |
| A 111 | – | + | + | légère | 6,5 | faible |
| B 222 | + | – | + | – | 6,0 | – |
| A 112 | – | + | + | – | 6,5 | – |
| A 13 | – | + | – | – | 6,0 | faible |
| A 141 | – | – | + | – | 6,5 | faible |
| A 142 | – | + | + | – | 6,0 | faible |
| B 211 | – | + | + | – | 6,5 | forte |
| B 212 | – | – | + | + | 6,0 | – |
| B 221 | – | + | + | – | 6,5 | – |
| C 211 | – | + | – | – | 6,0 | – |
| R 21 | – | + | + | – | 7,5 | – |
| R 22 | – | + | + | – | 6,0 | – |
| R 311 | – | + | + | – | 6,0 | faible |
| R 312 | – | – | + | légère | 6,0 | – |
| R 331 | – | + | – | + | 6,0 | – |

Toutes les souches donnent de l'ammoniaque en fin de culture sur nitrates; en outre, elles donnent des nitrites sauf les souches B 221, B 211, B 212 et C 211. La souche B 222 donne un dégagement gazeux à partir des nitrates.

La souche R 332 appartient au genre Bacillus, mais présente une faible activité protéolytique. Les souches R 340 et R 341 sont voisines du genre Bactiridium au sens de Prévot. Les autres souches sont asporulées. La souche A 111 est un Micrococcus. Toutes les autres souches sont voisines du genre Brevibacterium au sens de Bergey. Il est à noter que la souche B 222 est très voisine de Brevibacterium imperiale.

Parmi les souches utilisables possédant une amidase L stéréospécifique et une nitrilase générale, il faut citer plus particulièrement la souche $A_4$ déposée sous le n° LMD 79.2 au Centraalbureau voor Schimmelcultures en date du 6 mars 1979.

Le procédé selon la présente invention permet donc d'obtenir le L-$\alpha$-amino-acide ainsi qu'un D-$\alpha$-amino-amide non hydrolysé. Ce dernier amide peut être racémisé puis retraité selon le procédé de l'invention afin de préparer une nouvelle quantité de L-$\alpha$-amino-acide. La racémisation peut être conduite par tout procédé connu, en particulier par chauffage en présence de cétone ou d'acide.

Comme on a, d'autre part, constaté que les différentes souches mentionnées précédemment possédant une amidase générale ne possédaient pas de racémase, si l'on traite le D-$\alpha$-amino-acide obtenu par ces souches on obtient directement le D-$\alpha$-aminoacide correspondant.

L'invention concerne également l'application du D-$\alpha$-amino-amide obtenu à la préparation de D-$\alpha$-amino-acide correspondant par hydrolyse en présence d'un agent contenant une amidase

générale tel que les souches décirtes précédemment.

Le procédé selon la présente invention permet donc de préparer à l'état pur les deux stéréoisomères bien isolés.

Bien entendu, bien qu'il soit préférable d'utiliser les bactéries, comme les procédés selon la présente invention ne nécessitent pas la croissance des bactéries, il est possible également d'utiliser les préparations acellulaires provenant de ces bactéries que peuvent se présenter, par exemple, sous la forme d'enzymes fixées ou sous la forme de cellules branchées ou fixées sur un support ou absorbées par lui, ce qui peut, dans certains cas, faciliter leur manipulation.

Le procédé selon la présente invention permet donc d'obtenir les acides α-aminés naturels à activité optique comme par exemple l'α-alanin, la méthionine, le phénylalanine, la leucine, la valine.

Il est également possible, bien sûr, de synthétiser des acides α-aminés sous leur forme D optiquement purs.

Enfin, il est possible d'obtenir des acides α-aminés à activité optique qui ne figurent pas parmi les constituants habituels des protéines.

Bien que les paramètres particuliers de mise en oeuvre du procédé selon la présente invention ne soient pas critiques, il est intéressant d'opérer à température et pression ambiantes.

Le procédé est conduit de préférence en milieu aqueux. Bien que dans certains cas la faible solubilité dans l'eau du nitrile pose un problème, ceci n'entrave pas notablement l'activité nitrilasique ou L-amidasique des bactéries utilisables dans le procédé selon l'invention.

Un avantage du procédé selon l'invention est qu'il est possible de recycler les bactéries qui sont encore actives à la fin du procédé.

Enfin, le pH du milieu est de préférence compris entre 6 et 9.

Les exemples suivants sont donnée afin d'illustrer la mise en oeuvre du procédé selon la présente invention sans pour autant, bien entendu, la limiter en aucune façon.

## Exemple 1

### Préparation du mutant $A_4$

On effectue un étalement en masse d'une culture de la souche CBS 717.73 sur »Yeast natural broth« (YNB)-acétate d'ammonium 0,5%-fluoro-acétamide 1% gélosé (pH 6,5). Au bout de 8 à 10 jours des colonies résistantes apparaissent; elles sont prélevées sur un milieu YNB-acétate d'ammonium 0,5% sans fluoroacétamide (pH 6,5). Leur résistance au fluoro-acétamide est ensuite testée en milieu liquide comparativement à la souche sauvage. La souche mutante est ensuite testée en milieu liquide comparativement à la souche sauvage. La souche mutante est ensuite étalée sur YNB-acétate d'ammonium 0,5% (pH 6,5) pour vérifier l'homogénéité et l'on effectue un »replica-plating« sur YNB-acétamide 0,5% (pH 6,5) et YNB-acétate d'ammonium 0,5%-fluoro-acétamide 1% (pH 6,5). Plusieurs mutants défectifs ont été ainsi criblés à partir de la souche R 312 (CBS 717.73).

Ils sont stables en multiplication végétative. Une seule souche mutante a été utilisée dans les applications: la souche $A_4$ (collection de la chaire de génétique et microbiologie).

## Exemple 2

### Préparation de L-méthionine à partir de chlorhydrate d'α-amino-γ-méthylthiobutyronitrile DL

Une solution à 6% de chlorhydrate d'α-amino-γ-méthylthiobutyronitrile (pH compris entre 6,5 et 8,5) est traitée par des bactéries de la souche $A_4$ à la concentration d'environ 30 g de matière sèche par litre. La transformation est quantitative en L-méthionine (50%) et en D-amide correspondant (50%) en 2 à 3 heures. Les produits sont séparés par les techniques connues. L'amide D est racémisé et peut être recyclé.

## Exemple 3

### Préparation de D-méthionine à partir du chlorhydrate d'α-amino-γ-méthylthiobutyronitrile DL

Le traitement commence dans les mêmes conditions que ci-dessus. Après séparation, l'α-amino-γ-méthylthiobutyramide D est traité par des bactéries de la souche R 312 (CBS 717.73) dans les mêmes conditions. On obtient ainsi la D-méthionine. L'hydrolyse de l'α-amino-γ-méthylthiobutyronitrile permet donc d'obtenir, bien séparées, de la L-méthionine et de la D-méthionine.

Exemple 4

Préparation de L-méthionine à partir du chlorhydrate d'α-amino-γ-méthylthiobutyramide DL

Le traitement d'une solution à 6% de chlorhydrate d'α-amino-γ-méthylthiobutyramide DL dans l'eau (pH 6,5 à 8,5) est effectué par une suspension de cellules de la souche $A_4$ à 20 à 40 g par litre de matière sèche. On obtient quantitativement de la L-méthionine (50%) et de la d-méthionamide (50%). Cet amide D peut être, soit hydrolysé en D-méthionine par action de la souche R 312 (CBS 717.73), soit racémisé et recyclé en vue de la préparation de L-méthionine.

Exemple 5

Préparation de L-phénylalanine à partir de chlorhydrate d'amino-2-phényl-3-propionitrile DL

Une solution à 5% de chlorhydrate d'amino-2-phényl-3-propionitrile (pH compris entre 6,5 et 8,5) est traitée par des bactéries de la souche $A_4$ à la concentration de 20 à 40 g de matière sèche par litre. La transformation est quantitative en L-phényalanine (50%) et en D-amide correspondant (50%) en 2 à 3 heures. Les produits sont séparés par les techniques connues. L'amide D est racémisé et peut être recyclé.

Exemple 6

Préparation de D-phénylalanine à partir de chlorhydrate d'amino-2-phényl-3-propionitrile DL

Le traitement commence dans les mêmes conditions que ci-dessus. Après séparation, l'amino-2-phényl-3-propionamide D est traité par des bactéries de la souche R 312 (CBS 717.73) dans les mêmes conditions. On obtient ainsi la D-phénylalanine. L'hydrolyse de l'amino-2-phényl-3-propionitrile permet donc d'obtenir, bien séparées, de la L-phénylalanine et de la D-phénylalanine.

Exemple 7

Préparation de L-phénylalanine à partir du chlorhydrate d'amino-2-phényl-3-propionamide DL

Le traitement d'une solution à 5% de chlorhydrate d'amino-2-phényl-3-propionamide dans l'eau (pH compris entre 6,5 et 8,5) est effectué par une suspension de cellules de la souche $A_4$ à 20 à 40 g de matière sèche par litre. On obtient quantitativement de la L-phénylalanine (50%) et du D-amide correspondant (50%). Cet amide D peut être, soit hydrolysé en D-phénylalanine par action de la souche R 312 (CBS 717.73), soit racémisé et recyclé en vue de la préparation de L-phényl-alanine.

Exemple 8

Préparation de L-α-alanine à partir de chlorhydrate d'α-amino-propionitrile DL

Une solution à 6% de chlorhydrate d'α-amino-propionitrile dans l'eau (pH compris entre 6,5 et 8,5) est traitée par des bactéries de la souche $A_4$ à la concentration de 20 à 40 g de matière sèche par litre. La transformation est quantitative en L-α-alanine (50%) et en D-α-aminopropionamide (50%) en 2 à 3 heures. Les produits sont séparés par les techniques connues. L'amide D est racémisé et peut être recyclé.

Exemple 9

Préparation de D-α-alanine à partir de chlorhydrate d'α-aminopropionitrile DL

Le traitement commence dans les mêmes conditions que ci-dessus. Après séparation, l'α-amino-propionamide D est traité par des bactéries de la souche R 312 (CBS 717.73) dans les mêmes

conditions. On obtient ainsi la D-α-alanine. L'hydrolyse de l'α-aminopropionitrile permet donc d'obtenir, bien séparées, de la L-α-alanine et de la D-α-alanine.

Exemple 10

Préparation de L-α-alanine à partir du chlorhydrate d'α-aminopropionamide DL

Le traitement d'une solution à 6% de chlorhydrate d'α-aminopropionamide dans l'eau (pH compris entre 6,5 et 8,5) peu être effectué par une suspension de cellules de la souche $A_4$ à 20 à 40 g de matière sèche par litre. On obtient quantitativement de la L-α-alanine (50%) et du D-α-aminopropionamide (50%). Cet amide D peut être, soit hydrolysé en D-α-alanine par action de la souche R 312 (CBS 717.73), soit racémisé et recyclé en vue de la préparation de L-α-alanine.

**Revendications**

1. Procédé de préparation de L-α-amino-acide à partir de l'α-amino-nitrile racémique correspondant sous forme libre ou sous forme de sel, caractérisé en ce que l'on hydrolyse, en une seule étape, en milieu liquide, l'α-amino-nitrile racémique correspondant sous forme libre ou sous forme de sel à l'aide d'une bactérie possédant une nitrilase générale et une amidase L-stéréospécifique ou une préparation acellulaire extraite de cette bactérie et en ce que l'on sépare le L-α-amino-acide obtenu du D-α-amino-amide non hydrolysé.

2. Procédé selon la revendication 1, caractérisé en ce que la souche bactérienne mise en œuvre appartient aux genres Bacillus, Bacteridium au sens de Prévot, Micrococcus ou Brevibacterium au sens de Bergey.

3. Procédé selon l'une des revendications 1 et 2, caractérisé en ce que la souche utilisée est un mutant des souches R 332, R 340, R 341, A 111, B 222, A 112, A 13, A 141, A 142, B 211, B 212, B 221, C 211, R 21, R 22, R 311, R 312, R 331, déposées à la Chaire de Génétique de l'Ecole Nationale Supérieure Agronomique de Montpellier et présentant les caractéristiques morphologiques et physiologiques décrites dans les tableaux I et II de la description.

4. Procédé selon l'une des revendications 1 et 2, caractérisé en ce que la souche utilisée est un mutant des souches déposées au Centraal Bureau voor Schimmelcultures à Delft sous les n° C 211 CBS 499.74, R 312 CBS 717.73, B 222 CBS 498.74, A 111 CBS 497.74, R 341 CBS 496.74, R 340 CBS 495.74, R 332 CBS 494.74.

5. Procédé selon la revendication 1, caractérisé en ce que la couche bactérienne mise en œuvre est la souche $A_4$ déposée sous le n° LMD 79.2.

6. Procédé selon l'une des revendications 1 à 5, caractérisé en ce que l'hydrolyse est conduite à un pH compris entre 6 et 9.

7. Procédé selon l'une des revendications 1 à 6, caractérisé en ce que l'hydrolyse est conduite en milieu aqueux.

8. Procédé selon l'une des revendications 1 à 7, caractérisé en ce que l'α-amino-amide racémique est préparé par racémisation du D-α-amino-amide non hydrolysé d'une préparation précédente.

9. Application du D-α-amino-amide obtenu selon la revendication 1 à la préparation de D-α-amino-acide par hydrolyse en présence d'un agent contenant une amidase générale.

**Patentansprüche**

1. Verfahren zur Herstellung von L-α-Aminosäure aus dem entsprechenden racemischen α-Aminonitril in freier oder Salzform, dadurch gekennzeichnet, daß man in einer Stufe in wäßrigem Milieu das entsprechende racemische α-Aminotril in freier oder Salzform mittels Bakterien oder eines nicht zellförmigen Extraktes dieser Bakterien mit allgemeiner Nitrilase und L-stereospezifischer Amidase hydrolisiert, und man die erhaltene L-α-Aminosäure von dem nicht hydrolisierten L-α-Aminoamid abtrennt.

2. Verfahren nach Anspruch 1, dadurch gekennzeichnet, daß man einen Bakterienstamm der Gattung Bacillus, Bacteridium nach Prévot, Micrococcus oder Brevibacterium nach Bergey verwendet.

3. Verfahren nach einem der Ansprüche 1 und 2, dadurch gekennzeichnet, daß man als Stamm einen Mutanten der Stämme R 332, R 340, R 341, A 111, B 222, A 112, A 13, A 141, A 142, B 211, B 212, B 221, C 211, R 21, R 22, R 311, R 312, R 331 verwendet, die am Chaire de Génétique de l'Ecole Nationale Supérieure Agronomique de Montpellier hinterlegt sind, und die morphologischen und physiologischen Kennzeichen gemäß Tabelle I und II der Beschreibung aufweisen.

4. Verfahren nach einem der Ansprüche 1 und 2, dadurch gekennzeichnet, daß man als Stamm einen Mutanten der am Centraal Bureau voor Schimmelcultures in Delft unter den Nummern C 211 CBS

499.74, R 312 CBS 717.73, B 222 CBS 498.74, A 111 CBS 497.74, R 341 CBS 496.74, R 340 CBS 495.74, R 332 CBS 494.74 hinterlegten Stämme verwendet.

5. Verfahren nach Anspruch 1, dadurch gekennzeichnet, daß man als Bakterienstamm den Stamm $A_4$, der unter der Nummer LMD 79.2 hinterlegt wurde, verwendet.

6. Verfahren nach einem der Ansprüche 1 bis 5, dadurch gekennzeichnet, daß man die Hydrolyse bei einem pH-Wert zwischen 6 und 9 durchführt.

7. Verfahren nach einem der Ansprüche 1 bis 6, dadurch gekennzeichnet, daß man die Hydrolyse in wäßrigem Milieu durchführt.

8. Verfahren nach einem der Ansprüche 1 bis 7, dadurch gekennzeichnet, daß das racemische α-Aminoamid durch Racemisierung von nicht hydrolisiertem D-α-Aminoamid aus einem vorhergehenden Herstellungsverfahren hergestellt wird.

9. Verwendung des D-α-Aminoamids gemäß Anspruch 1 zur Herstellung von D-α-Aminosäure durch Hydrolyse in Gegenwart eines allgemeine Amidase enthaltenden Mittels.

## Claims

1. A process for the preparation of L-α-amino acid from the corresponding racemic α-amino-nitrile in free form or in salt form, characterised in that the corresponding racemic α-amino-nitrile is hydrolysed in a single stage, in a liquid medium, in free form or in salt form using a bacterium having a general nitrilase and an L-stereospecific amidase or an acellular preparation extracted from this bacterium, and the L-α-amino acid which is obtained is separated from the unhydrolysed D-α-amino-amide.

2. A process according to claim 1, characterised in that the bacterial strain which is used belongs to the genera Bacillus, Bacteridium in the meaning of Prévot, Micrococcus or Brevibacterium in the meaning of Bergey.

3. A process according to one of claims 1 and 2, characterised in that the strain which is used is a mutant of the strains R 332, R 340, R 341, A 111, B 222, A 112, A 13, A 141, A 142, B 211, B 212, B 221, C 211, R 21, R 22, R 311, R 312 and R 331, deposited at the Chaire de Génétique de l'Ecole Nationale Supérieure agronomique de Montpellier and having morphological and physiological characteristics described in Tables I and II of the description.

4. A process according to one of claims 1 and 2, characterised in that the strain which is used is a mutant of the strains deposited at the Centraal Bureau voor Schimmelcultures at Delft under numbers C 211 CBS 399.74, R 312 CBS 717.73, B 222 CBS 498.74, A 111 CBS 497.74, R 341 CBS 496.74, R 340 CBS 495.74 and R 332 CBS 494.74.

5. A process according to claim 1, characterised in that the bacterial strain which is used is strain $A_4$ deposited under No. LMD 79.2

6. A process according to one of claims 1 to 5, characterised in that hydrolysis is carried out at a pH between 6 and 9.

7. A process according to one of claims 1 to 6, characterised in that hydrolysis is carried out in an aqueous medium.

8. A process according to one of claims 1 to 7, characterised in that the racemic α-amino-amide is prepared by the racemization of the unhydrolysed D-α-amino-amide from a preceding preparation.

9. The use of D-α-amino-amide obtained according to claim 1 for the preparation of D-α-amino-acid by hydrolysis in the presence of an agent containing a general amidase.